# EUROPEAN PATENT APPLICATION

(11) **EP 3 721 917 A2**
(43) Date of publication of application: **14.10.2020**
(21) Application number: 20168630.0
(22) Date of filing: 08.04.2020
(51) Int. Cl.: A61M 1/00

(54) **VACUUM DRIVEN SUCTION AND IRRIGATION SYSTEM**

(30) Priority: 09.04.2019 US 201962831265 P; 13.03.2020 US 202016818569
(71) Applicant: Covidien LP, Mansfield, MA 02048 (US)
(72) Inventor: HEJMOWSKI, Tomasz, Derby, CT Connecticut 06418 (US); PRIBANIC, Russell, Roxbury, CT Connecticut 06783 (US); HORTON, Kenneth W., South Glastonbury, CT Connecticut 06073 (US); FULLER, Bob, Arvada, CO Colorado 80002 (US)
(74) Representative: Maschio, Antonio

(57) **Abstract**

A vacuum assisted suction and irrigation system includes a suction and irrigation wand, an irrigation fluid supply, a vacuum source, and a dual impeller fluid pump. The vacuum source is connected to a suction valve of the suction and irrigation wand to provide suction within the suction and irrigation wand. The irrigation fluid supply is connected to the suction and irrigation wand via the dual impeller fluid pump to supply pressurized irrigation fluid to the suction and irrigation wand. The vacuum source is connected to the fluid pump to pressurize the irrigation fluid being delivered to the suction and irrigation wand.

## Description

### CROSS-REFERENCE TO RELATED APPLICATION

The present application claims the benefit of and priority to U.S. Provisional Patent Application Ser. No. 62/831,265 filed on April 9, 2019, the entire content of which is incorporated herein by reference.

### BACKGROUND

### 1. Technical Description

The present disclosure is directed to a suction and irrigation system and, more particularly, to a vacuum assisted suction and irrigation system.

### 2. Background of Related Art

Suction and irrigation systems that include a surgical instrument to provide both suction and irrigation to a surgical site are well known. Typically, such systems include a source of vacuum that is coupled to the surgical instrument to provide suction and an electric, e.g., battery powered, pump to deliver irrigation fluid to the surgical instrument. There are two categories of pumps including disposable pumps and reusable pumps. The disposable pumps are typically powered by direct current (DC), whereas the reusable pumps are powered with alternating current or bottled gas. Thus, two types of power sources are required to use the surgical instrument. The requirement of two power sources results in added cost, complexity, and clutter.

A continuing need exists in the surgical arts for a low cost, easy to use suction and irrigation system that can operate using a single energy source, i.e., vacuum, that is already available in an operating room.

### SUMMARY

One aspect of the present disclosure is directed to a vacuum assisted suction and irrigation system that includes a suction and irrigation wand, an irrigation fluid supply, a vacuum source, and a dual impeller pump. The suction and irrigation wand includes a proximal body portion that supports suction and irrigation valves and a distal body portion that defines a fluid channel. The irrigation fluid supply is connected to the irrigation valve of the suction and irrigation wand and the vacuum source is connected to the suction valve of the suction and irrigation wand. The irrigation valve is actuable to deliver irrigation fluid to the fluid channel of the suction and irrigation wand and the suction valve is actuable to draw a vacuum within the fluid channel of the suction and irrigation wand. The dual impeller pump is configured to deliver pressurized irrigation fluid from the irrigation fluid supply to the suction and irrigation wand and includes a pump impeller and a turbine wheel. The dual impeller pump is connected to the vacuum source to drive the pump impeller and the turbine wheel.

Another aspect of the disclosure is directed to a vacuum assisted suction and irrigation system that includes a suction and irrigation wand, an irrigation fluid supply, a vacuum source, and a dual impeller pump. The suction and irrigation wand includes a proximal body portion that supports a suction valve and an irrigation valve and a distal body portion that defines a fluid channel. The irrigation fluid supply is connected to the irrigation valve of the suction and irrigation wand. The irrigation valve is actuable to deliver irrigation fluid to the fluid channel of the suction and irrigation wand. The vacuum source is connected to the suction valve of the suction and irrigation wand. The suction valve is actuable to draw a vacuum within the fluid channel. The dual impeller pump is connected to the irrigation fluid supply and to the vacuum source to deliver pressurized irrigation fluid from the irrigation fluid supply to the suction and irrigation wand. The dual impeller pump includes a pump impeller and a turbine wheel. The vacuum source drives the turbine wheel to drive the pump impeller. The irrigation fluid supply is positioned above the first pump chamber such that irrigation fluid from the irrigation fluid supply forces air from the first pump chamber to prime the dual impeller pump.

Another aspect of the disclosure is directed to a vacuum assisted suction and irrigation system that includes a suction and irrigation wand, an irrigation fluid supply, a vacuum source, and a dual impeller pump. The suction and irrigation wand includes a proximal body portion that supports a suction valve and an irrigation valve and a distal body portion that defines a fluid channel. The irrigation fluid supply is connected to the irrigation valve of the suction and irrigation wand. The irrigation valve is actuable to deliver irrigation fluid to the fluid channel of the suction and irrigation wand. The vacuum source is connected to the suction valve of the suction and irrigation wand. The suction valve is actuable to draw a vacuum within the fluid channel. The dual impeller pump is configured to deliver pressurized irrigation fluid from the irrigation fluid supply to the suction and irrigation wand. The dual impeller pump includes a housing that defines first and second pump chambers, a pump impeller, and a turbine wheel. The pump impeller is positioned within the first pump chamber and the turbine wheel is positioned within the second pump chamber. The dual impeller pump is connected to the vacuum source to drive the pump impeller and the turbine wheel. The first pump chamber includes a fluid inlet port and a fluid outlet port, and the second pump chamber includes an air inlet port and an air outlet port. The air inlet port defines an inlet channel that communicates with the second pump chamber. The inlet channel has a diameter that converges towards the second pump chamber.

In embodiments, the dual impeller pump includes a housing defining a first pump chamber and a second pump chamber. The pump impeller is positioned within the first pump chamber and the turbine wheel is positioned within the second pump chamber.

In some embodiments, the first pump chamber includes a fluid inlet port and a fluid outlet port and the second pump chamber includes an air inlet port and an air outlet port.

In certain embodiments, the fluid inlet port communicates with the irrigation fluid supply and the air outlet port communicates with the vacuum source.

In embodiments, the fluid outlet port communicates with the suction and irrigation wand and the air inlet port communicates with atmosphere.

In some embodiments, the air inlet port defines an inlet channel that communicates with the second pump chamber and has a diameter that converges towards the second pump chamber.

In certain embodiments, the pump impeller is connected to the turbine wheel by a pump shaft such that rotation of the turbine wheel within the second pump chamber causes rotation of the pump impeller within the first pump chamber. Alternately, the pump impeller can be connected to the turbine wheel by a magnetic coupling such that rotation of the turbine wheel within the second pump chamber causes rotation of the pump impeller within the first pump chamber.

In embodiments, the housing includes a first housing portion and a second housing portion that is coupled to the first housing portion.

In some embodiments, the first housing portion defines the first pump chamber and the second housing portion defines the second pump chamber.

In certain embodiments, the first housing portion includes the fluid inlet port and the fluid outlet port and the second housing portion includes the air inlet port and the air outlet port.

In embodiments, the second housing portion includes a first end and a second end, and the first end of the second housing portion is configured to be received within the first housing portion.

In some embodiments, the dual impeller pump includes an O-ring and the first end of the second housing portion defines an annular groove, wherein the O-ring is received within the annular groove to provide a seal between the first housing portion and the second housing portion.

In certain embodiments, the dual impeller pump includes an end cap and the second housing portion includes a second end opposite to the first end that defines an opening, wherein the end cap is positioned within the opening at the second end of the second housing portion to close the opening.

In embodiments, the pump shaft has a first end and a second end and the second housing portion defines a through bore that connects the first pump chamber to the second pump chamber, wherein the pump shaft is positioned within the through bore such that the first end of the pump shaft is positioned within the first pump chamber and the second end of the pump shaft is positioned within the second pump chamber.

In some embodiments, the first end of the pump shaft is secured to the pump impeller and the second end of the pump shaft is secured to the turbine wheel.

In certain embodiments, the dual impeller pump includes a bearing that is positioned about the pump shaft within the through bore.

In embodiments, the second housing portion includes an annular inner wall and the turbine wheel defines a cylindrical bore. The annular inner wall is received within the cylindrical bore to rotatably support the turbine wheel on the second housing portion.

In some embodiments, the dual impeller pump includes a bushing positioned about the annular inner wall of the second housing portion between the turbine wheel and the annular inner wall.

### BRIEF DESCRIPTION OF THE DRAWINGS

Various embodiments of the presently disclosed vacuum assisted suction and irrigation system are described herein below with reference to the drawings, wherein:
FIG. 1 is a side perspective, schematic view of one exemplary embodiment of the presently disclosed vacuum assisted suction and irrigation system;
FIG. 2 is a side perspective view of a fluid pump of the vacuum assisted suction and irrigation system shown in FIG.1;
FIG. 3 is an exploded perspective view of the fluid pump of FIG. 2;
FIG. 4 is a cross-sectional view taken along section line 4-4 of FIG. 2;
FIG. 5 is a cross-sectional view taken along section line 5-5 of FIG. 2;
FIG. 6 is a cross-sectional view taken along section line 6-6 of FIG. 2; and
FIG. 7 is a cross-sectional view taken along section line 7-7 of FIG. 2.

### DETAILED DESCRIPTION OF EMBODIMENTS

The presently disclosed vacuum assisted suction and irrigation system will now be described in detail with reference to the drawings in which like reference numerals designate identical or corresponding elements in each of the several views. However, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

In this description, the term "proximal" is used generally to refer to that portion of the device that is closer to a clinician, while the term "distal" is used generally to refer to that portion of the device that is farther from the clinician. In addition, the term "clinician" is used generally to refer to medical personnel including doctors, nurses, and support personnel.

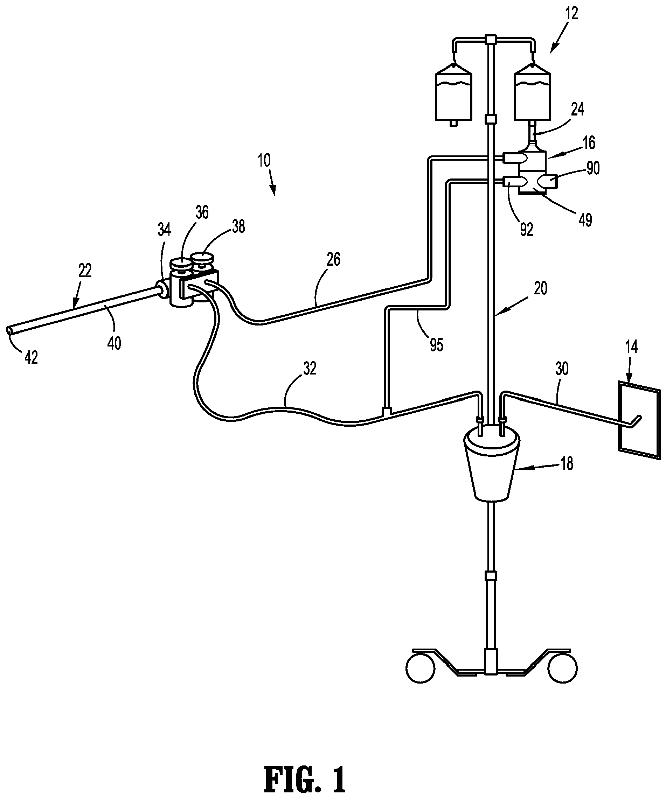

An exemplary embodiment of the presently disclosed vacuum assisted suction and irrigation system is shown generally in FIG. 1 as system 10. The system 10 includes an irrigation fluid supply 12, a vacuum source 14, a fluid pump 16, a suction container 18, and a suction and irrigation wand 22. In embodiments, the system 10 is supported on a wheeled stand 20 although the use of other stationary and movable support devices to support the system 10 is envisioned. The components of the device 10 are fluidly coupled to each other with fluid conduits 24-32 as described in further detail below.

The suction and irrigation wand 22 includes a proximal body portion 34 that supports a suction valve 36 and an irrigation valve 38, and a distal body portion 40 that defines a fluid channel 42 for receiving or dispensing fluid depending on which valve of the suction valve 36 and irrigation valve 38 is actuated. In embodiments, the proximal body portion 34 of the suction and irrigation wand 22 is connected to the irrigation fluid supply 12 by the fluid conduits 24 and 26 such that upon actuation of the irrigation valve 38, irrigation fluid is delivered to the fluid channel 42 of the distal body portion 40 of the suction and irrigation wand 22. Similarly, the proximal body portion 34 of the suction and irrigation wand 22 is connected to the vacuum source 14 by the fluid conduits 30 and 32 via the suction container 18 such that upon actuation of the suction valve 36, a vacuum is created in the fluid channel 42 of the distal body portion 40 of the suction and irrigation wand 22 to draw fluid into the fluid channel 42. In embodiments, the suction and irrigation valves 36, 38 may be trumpet type valves although the use of other valve types is envisioned.

In embodiments, the suction and irrigation wand 22 communicates with the suction container 18 via the fluid conduit 32 and the suction container 18 communicates with the vacuum source 14 via the fluid conduit 30. As such, a vacuum is maintained within the suction container 18 by the vacuum source 14. Thus, when the suction valve 36 is actuated, the fluid channel 42 communicates with the suction container 18 to create a vacuum within the fluid channel 42. All materials drawn into the channel 42 of the suction and irrigation wand 22, such as blood, tissue, saline, etc., are delivered to and collected within the suction container 18.

The irrigation fluid supply 12 is coupled to the fluid pump 16 by fluid conduit 24 and the fluid pump 16 is coupled to the suction and irrigation wand 22 by the fluid conduit 26 such that when the irrigation valve 38 is actuated, fluid from the irrigation fluid supply 12 is delivered to the fluid channel 42 of the suction and irrigation wand 22 via the fluid pump 16. In some embodiments, the irrigation fluid supply 12 can be connected directly to the suction and irrigation wand 22 and other mechanisms can be provided to pressurize the irrigation fluid supply 12.

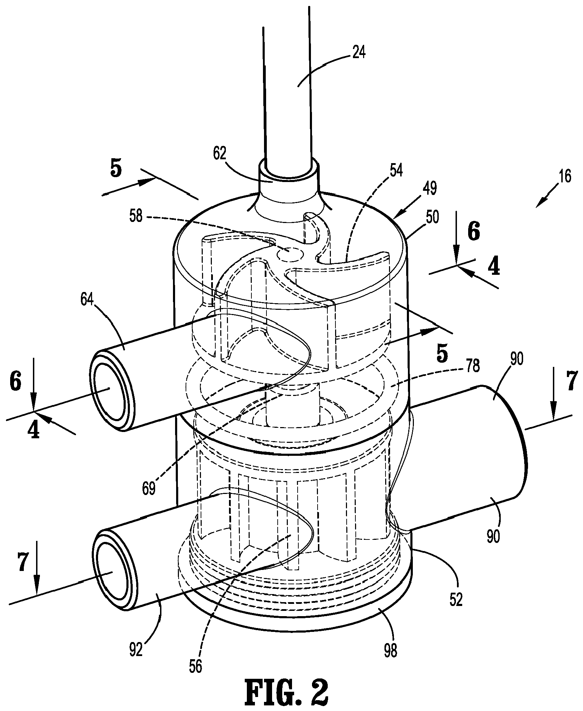

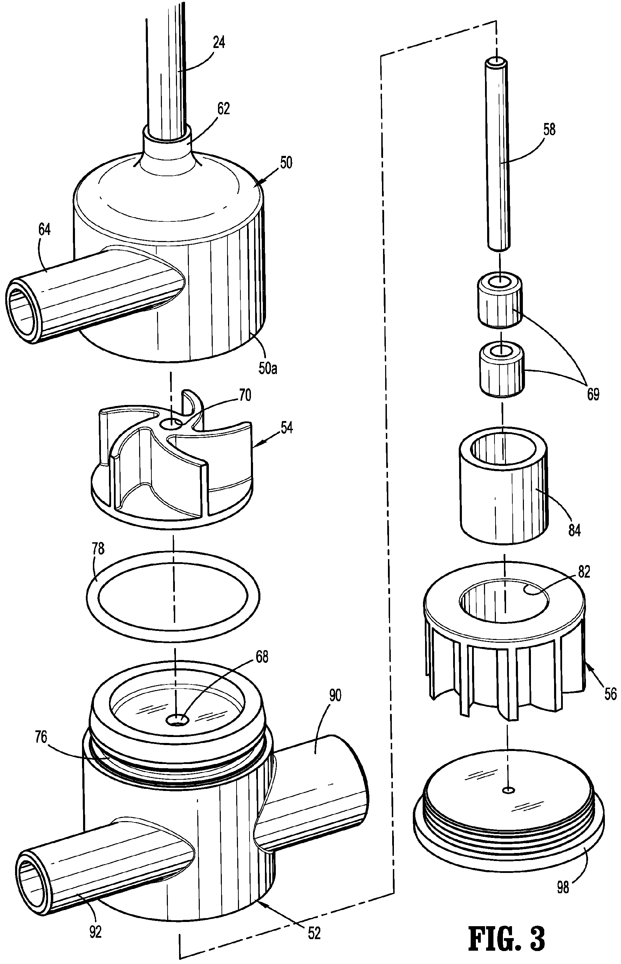

FIGS. 2-7 illustrate an exemplary embodiment of the fluid pump16. Referring initially to FIG. 2, in embodiments, the fluid pump 16 is a dual impeller type pump and includes a housing 49 having a first housing portion 50 and a second housing portion 52, a pump impeller 54, a turbine wheel 56, and a pump shaft 58. The first housing portion 50 defines a first pump chamber 60 and includes an inlet port 62 and an outlet port 64. The inlet port 62 communicates with the irrigation fluid supply 12 via the fluid conduit 24. In embodiments, the irrigation fluid supply 12 is positioned above the first pump chamber 60 such that irrigation fluid from the irrigation fluid supply 12 forces air from the first pump chamber 60 to prime the fluid pump 16. Although not shown, the inlet port 62 may include a valve to control the flow of irrigation fluid into the first pump chamber 60. The outlet port 64 is connected to the suction and irrigation wand 22 by the fluid conduit 26.

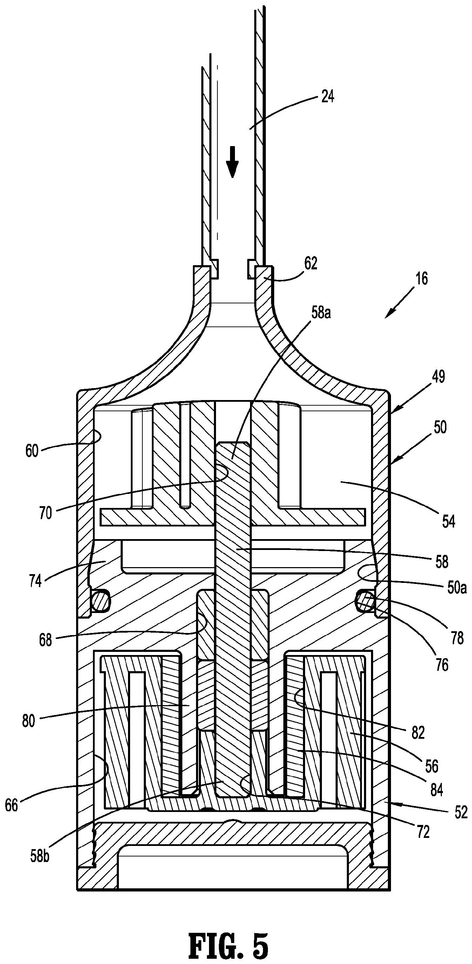

The second housing portion 52 defines a second pump chamber 66 and a through bore 68 that communicates with the first pump chamber 60. The pump shaft 58 extends through and is rotatable within the through bore 68 (FIG. 5). In embodiments, the pump shaft 58 is supported within the through bore 68 (FIG. 5) by bearings 69. The pump shaft 58 has a first end 58a disposed within the first pump chamber 60 and a second end 58b disposed within the second pump chamber 66. The pump impeller 54 is coupled to the first end 58a of the pump shaft 58 within the first pump chamber 60 and the turbine wheel 56 is coupled to the second end 58b of the pump shaft 58 within the second pump chamber 66. In embodiments, the pump impeller 54 defines a central bore 70 that receives the first end 58a of the pump shaft 58 and the turbine wheel 56 defines a central bore 72 that receives the second end 58b of the pump shaft 58. In some embodiments, the first and second ends 58a, 58b of the pump shaft 58 are press fit into the central bores 70, 72 of the pump impeller 54 and turbine wheel 56, such that rotation of the turbine wheel 56 within the second pump chamber 60 causes corresponding rotation of the pump impeller 54 within the first pump chamber 60. Alternately, it is envisioned that the pump shaft 58 may be connected to the pump impeller 54 and the turbine wheel 56 using a variety of different coupling techniques such as pinning, welding, or the like.

Although not shown, the pump impeller 54 can be connected to the turbine wheel 56 by a magnetic coupling such that rotation of the turbine wheel 56 within the second pump chamber 66 causes rotation of the pump impeller 54 within the first pump chamber 60. Alternately the use other known coupling device are envisioned.

The dual impeller pump 16 shown in FIG. 5 is not necessarily shown to scale. For example, the through bore 68, the bearings 69, and shaft 58 may be smaller than shown to be more suitable for its intended use. In addition, the bearings 69 may be spaced further apart.

The second housing portion 52 includes a first end 74 that is dimensioned to be received within an open end 50a of the first housing portion 50. The first end 74 of the second housing portion 52 defines an annular groove 76 that receives an O-ring 78. When the first end of the second housing portion 52 is received within the open end 50a of the first housing portion 50, the O-ring 78 provides a seal between the first and second housing portions 50, 52. In embodiments, the first and second housing portions 50, 52 are fixedly secured together using adhesives, ultra-sonic welding or the like.

The second housing portion 52 also includes an annular inner wall 80 (FIG. 5) that extends about and partially defines the through bore 68 of the second housing portion 52. The turbine wheel 56 defines a cylindrical bore 82 that is formed about the central bore 72 and receives the annular inner wall 80 of the second housing portion 52 such that the turbine wheel 56 is rotatably supported about the annular inner wall 80 of the second housing portion 52. In embodiments, a bushing 84 is positioned about the annular inner wall 80 between the turbine wheel 56 and the annular inner wall 80 to rotatably support the turbine wheel 56 on the second housing portion 52.

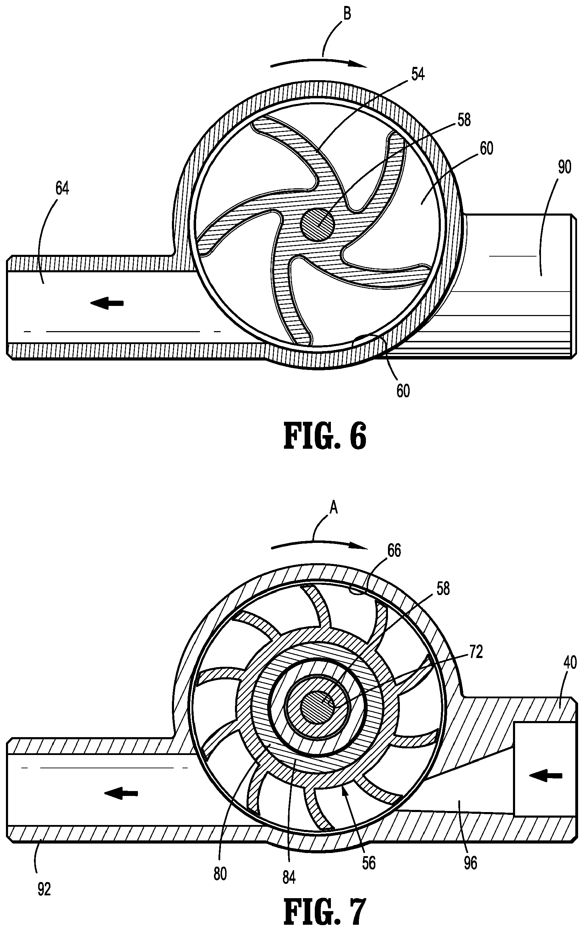

The second housing portion 52 further includes an air inlet port 90, an air outlet port 92 and an open end 94 (FIG. 1) opposite the first end 74 of the second housing portion 52. The air inlet port 90 communicates with atmosphere and the air outlet port 92 communicates with the vacuum source 14 via a fluid conduit 95. The air inlet port 90 defines an inlet channel 96 (FIG. 7) that converges in a direction towards the second pump chamber 66. In embodiments, the fluid conduit 95 communicates with the fluid conduit 32 which communicates with the suction container 18. Alternately, the fluid conduit 95 can communicate directly with the suction container 18 or directly with the vacuum source 14. The open end 94 of the second housing portion 52 is enclosed with an end cap 98 to enclose the second pump chamber 66.

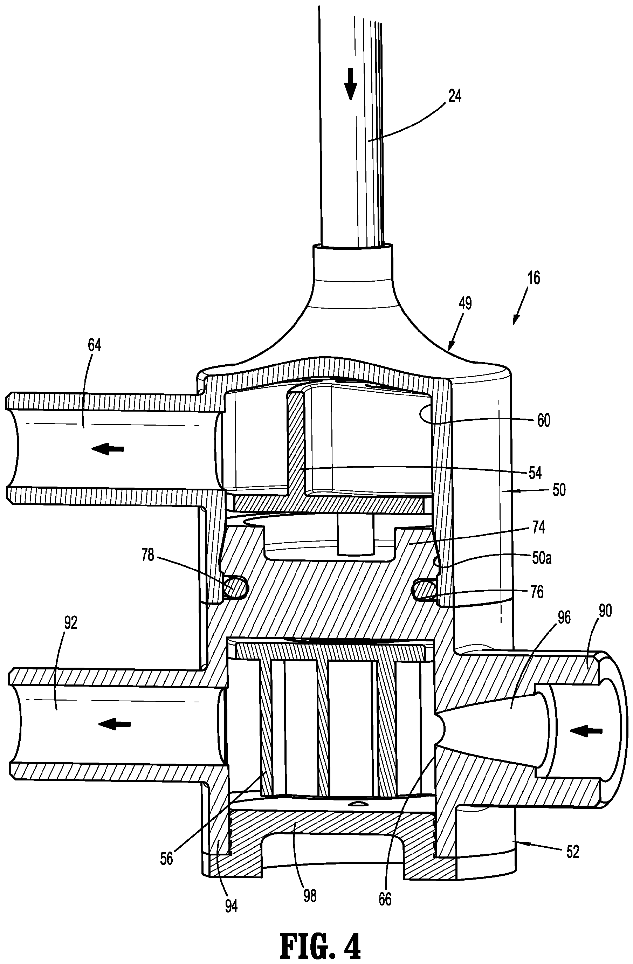

When a vacuum is applied to the air outlet port 92 of the pump 16 via the fluid conduit 95, the pressure within the second pump chamber 66 is reduced and air is drawn into the second pump chamber 66 through the air inlet port 90. As the air is drawn through the inlet channel 96, the air is compressed as the channel 96 (FIG. 4) converges towards the second pump chamber 66. As the compressed air exits the fluid channel 96 and enters the second pump chamber 66, the air expands and drives the turbine wheel 56 about the annular inner wall 80 of the second housing portion 52 in the direction indicate by arrow "A" in FIG. 7. Rotation of the turbine wheel 56 causes rotation of the pump shaft 58 which in turn causes rotation of the pump impeller 54 within the first pump chamber 60 in the direction indicated by arrow "B" in FIG. 6. As discussed above, the first pump chamber 60 is primed with irrigation fluid delivered from the irrigation fluid supply 12 via the inlet port 62 (FIG. 5) and fluid conduit 24. When the pump impeller 54 is rotated within the first pump chamber 60, the irrigation fluid is forced from the outlet port 64 of the first housing portion 50 into the fluid conduit 26 to deliver pressurized irrigation fluid to the suction and irrigation wand 22.

The vacuum assisted suction and irrigation system 10 (FIG. 1) operates to selectively deliver suction and/or irrigation fluid to a surgical site. More specifically, in use, a clinician grasps the proximal body portion 34 of the suction and irrigation wand 22 and manipulates the wand to position a distal end of the distal body portion 40 adjacent a surgical site (not shown). Although not shown, this may include inserting the distal body portion 40 of the suction and irrigation wand 22 through a cannula assembly to access the surgical site. If the clinician requires suction at the surgical site, e.g., to remove blood or debris, the clinician can actuate the suction valve 36 to draw material from the surgical site into the channel 42 of the suction and irrigation wand 22. All material withdrawn into the suction and irrigation wand 22 is delivered to the suction container 18 via the fluid conduit 32. If the clinician requires irrigation fluid, e.g., to improve visualization of the surgical site, the clinician can actuate the irrigation valve 38 to deliver fluid to the channel 42 of the distal body portion 40 of the suction and irrigation wand 22. In the presently disclosed system, vacuum from the vacuum source 14 which is available in an operating room drives the pump 16 to pressurize the irrigation fluid supplied to the suction and irrigation wand 22.

In embodiments, the components of the dual impeller pump, including the housing and the pump impeller 54 and turbine wheel 56 can be formed from plastic. In some embodiments, one or both of the pump impeller 54 and the turbine wheel 56 can be formed from a metal. In certain embodiments, the turbine wheel can be formed from metal or formed from plastic and weighted to increase the fluid flow generated by the dual impeller fluid pump 16.

Although the dual impeller pump 16 is shown vertically oriented with the pump impeller 54 and the first pump chamber 60 positioned vertically above the turbine wheel 56 in the second pump chamber 66, it is envisioned that the orientation of the dual impeller pump 16 may be horizontally oriented or inverted with minor variation to the system.

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described embodiments. Accordingly, the disclosure is not to be limited by what has been particularly shown and described, except as indicated by the appended claims.

The invention may be described by reference to the following numbered paragraphs:-
1. A vacuum assisted suction and irrigation system comprising:
   a suction and irrigation wand including a proximal body portion supporting a suction valve and an irrigation valve and a distal body portion defining a fluid channel;
   an irrigation fluid supply connected to the irrigation valve of the suction and irrigation wand, the irrigation valve being actuable to deliver irrigation fluid to the fluid channel of the suction and irrigation wand;
   a vacuum source connected to the suction valve of the suction and irrigation wand, the suction valve being actuable to draw a vacuum within the fluid channel; and
   a dual impeller pump configured to deliver pressurized irrigation fluid from the irrigation fluid supply to the suction and irrigation wand, the dual impeller pump including a pump impeller and a turbine wheel, wherein dual impeller pump is connected to the vacuum source to drive the pump impeller and the turbine wheel.
2. The suction and irrigation system of paragraph 1, wherein the dual impeller pump includes a housing defining first and second pump chambers, the pump impeller being positioned within the first pump chamber and the turbine wheel being positioned within the second pump chamber.
3. The suction and irrigation system of paragraph 2, wherein the first pump chamber includes a fluid inlet port and a fluid outlet port, and the second pump chamber includes an air inlet port and an air outlet port.
4. The suction and irrigation system of paragraph 3, wherein the fluid inlet port communicates with the irrigation fluid supply and the air outlet port communicates with the vacuum source.
5. The suction and irrigation system of paragraph 4, wherein the fluid outlet port communicates with the suction and irrigation wand and the air inlet port communicates with atmosphere.
6. The suction and irrigation system of paragraph 5, wherein the air inlet port defines an inlet channel that communicates with the second pump chamber, the inlet channel having a diameter that converges towards the second pump chamber.
7. The suction and irrigation system of paragraph 1, wherein the pump impeller is connected to the turbine wheel by a pump shaft such that rotation of the turbine wheel within the second pump chamber causes rotation of the pump impeller within the first pump chamber.
8. The suction and irrigation system of paragraph 7, wherein the housing includes a first housing portion and a second housing portion coupled to the first housing portion.
9. The suction and irrigation system of paragraph 8, wherein the first housing portion defines the first pump chamber and the second housing portion defines the second pump chamber.
10. The suction and irrigation system of paragraph 9, wherein the first housing portion includes the fluid inlet port and the fluid outlet port and the second housing portion includes the air inlet port and the air outlet port.
11. The suction and irrigation system of paragraph 8, wherein the second housing portion includes a first end and a second end, the first end being received within the first housing portion.
12. The suction and irrigation system of paragraph 11, wherein the dual impeller pump includes an O-ring and the first end of the second housing portion defines an annular groove, the O-ring being received within the annular groove to provide a seal between the first housing portion and the second housing portion.
13. The suction and irrigation system of paragraph 12, wherein the dual impeller pump includes an end cap and the second housing portion includes a second end opposite the first end that defines an opening, the end cap being positioned within the opening at the second end of the second housing portion to close the opening.
14. The suction and irrigation system of paragraph 7, wherein the pump shaft has a first end and a second end and the second housing portion defines a through bore that connects the first pump chamber to the second pump chamber, the pump shaft being positioned within the through bore such that the first end of the pump shaft is positioned within the first pump chamber and the second end of the pump shaft is positioned within the second pump chamber.
15. The suction and irrigation system of paragraph 14, wherein the first end of the pump shaft is secured to the pump impeller and the second end of the pump shaft is secured to the turbine wheel.
16. The suction and irrigation system of paragraph 15, wherein the dual impeller pump includes a bearing positioned about the pump shaft within the through bore.
17. The suction and irrigation system of paragraph 16, wherein the second housing portion includes an annular inner wall and the turbine wheel defines a cylindrical bore, the annular inner wall being received within the cylindrical bore to rotatably support the turbine wheel on the second housing portion.
18. The suction and irrigation system of paragraph 17, wherein the dual impeller pump includes a bushing positioned about the annular inner wall of the second housing portion between the turbine wheel and the annular inner wall.
19. A vacuum assisted suction and irrigation system comprising:
   a suction and irrigation wand including a proximal body portion supporting a suction valve and an irrigation valve and a distal body portion defining a fluid channel;
   an irrigation fluid supply connected to the irrigation valve of the suction and irrigation wand, the irrigation valve actuable to deliver irrigation fluid to the fluid channel of the suction and irrigation wand;
   a vacuum source connected to the suction valve of the suction and irrigation wand, the suction valve actuable to draw a vacuum within the fluid channel; and
   a dual impeller pump connected to the irrigation fluid supply and to the vacuum source to deliver pressurized irrigation fluid from the irrigation fluid supply to the suction and irrigation wand, the dual impeller pump including a pump impeller and a turbine wheel, the vacuum source driving the turbine wheel to drive the pump impeller, wherein the irrigation fluid supply is positioned above the first pump chamber such that irrigation fluid from the irrigation fluid supply forces air from the first pump chamber to prime the dual impeller pump.
20. A vacuum assisted suction and irrigation system comprising:
   a suction and irrigation wand including a proximal body portion supporting a suction valve and an irrigation valve and a distal body portion defining a fluid channel;
   an irrigation fluid supply connected to the irrigation valve of the suction and irrigation wand, the irrigation valve being actuable to deliver irrigation fluid to the fluid channel of the suction and irrigation wand;
   a vacuum source connected to the suction valve of the suction and irrigation wand, the suction valve being actuable to draw a vacuum within the fluid channel; and
   a dual impeller pump configured to deliver pressurized irrigation fluid from the irrigation fluid supply to the suction and irrigation wand, the dual impeller pump including a housing defining first and second pump chambers, a pump impeller, and a turbine wheel, the pump impeller positioned within the first pump chamber and the turbine wheel positioned within the second pump chamber, the dual impeller pump connected to the vacuum source to drive the pump impeller and the turbine wheel;
   wherein the first pump chamber includes a fluid inlet port and a fluid outlet port, and the second pump chamber includes an air inlet port and an air outlet port, the air inlet port defining an inlet channel that communicates with the second pump chamber, the inlet channel having a diameter that converges towards the second pump chamber.

## Claims

1. A vacuum assisted suction and irrigation system comprising:
a suction and irrigation wand including a proximal body portion supporting a suction valve and an irrigation valve and a distal body portion defining a fluid channel;
an irrigation fluid supply connected to the irrigation valve of the suction and irrigation wand, the irrigation valve being actuable to deliver irrigation fluid to the fluid channel of the suction and irrigation wand;
a vacuum source connected to the suction valve of the suction and irrigation wand, the suction valve being actuable to draw a vacuum within the fluid channel; and
a dual impeller pump configured to deliver pressurized irrigation fluid from the irrigation fluid supply to the suction and irrigation wand, the dual impeller pump including a pump impeller and a turbine wheel, wherein dual impeller pump is connected to the vacuum source to drive the pump impeller and the turbine wheel.

2. The suction and irrigation system of claim 1, wherein the dual impeller pump includes a housing defining first and second pump chambers, the pump impeller being positioned within the first pump chamber and the turbine wheel being positioned within the second pump chamber; preferably wherein the first pump chamber includes a fluid inlet port and a fluid outlet port, and the second pump chamber includes an air inlet port and an air outlet port.

3. The suction and irrigation system of claim 2, wherein the fluid inlet port communicates with the irrigation fluid supply and the air outlet port communicates with the vacuum source; preferably wherein the fluid outlet port communicates with the suction and irrigation wand and the air inlet port communicates with atmosphere.

4. The suction and irrigation system of claim 3, wherein the air inlet port defines an inlet channel that communicates with the second pump chamber, the inlet channel having a diameter that converges towards the second pump chamber.

5. The suction and irrigation system of any preceding claim, wherein the pump impeller is connected to the turbine wheel by a pump shaft such that rotation of the turbine wheel within the second pump chamber causes rotation of the pump impeller within the first pump chamber; preferably wherein the housing includes a first housing portion and a second housing portion coupled to the first housing portion.

6. The suction and irrigation system of claim 2, wherein the first housing portion defines the first pump chamber and the second housing portion defines the second pump chamber; preferably wherein the first housing portion includes the fluid inlet port and the fluid outlet port and the second housing portion includes the air inlet port and the air outlet port.

7. The suction and irrigation system of claim 6, wherein the second housing portion includes a first end and a second end, the first end being received within the first housing portion.

8. The suction and irrigation system of claim 7, wherein the dual impeller pump includes an O-ring and the first end of the second housing portion defines an annular groove, the O-ring being received within the annular groove to provide a seal between the first housing portion and the second housing portion.

9. The suction and irrigation system of claim 8, wherein the dual impeller pump includes an end cap and the second housing portion includes a second end opposite the first end that defines an opening, the end cap being positioned within the opening at the second end of the second housing portion to close the opening.

10. The suction and irrigation system of claim 5, wherein the pump shaft has a first end and a second end and the second housing portion defines a through bore that connects the first pump chamber to the second pump chamber, the pump shaft being positioned within the through bore such that the first end of the pump shaft is positioned within the first pump chamber and the second end of the pump shaft is positioned within the second pump chamber.

11. The suction and irrigation system of claim 10, wherein the first end of the pump shaft is secured to the pump impeller and the second end of the pump shaft is secured to the turbine wheel; preferably wherein the dual impeller pump includes a bearing positioned about the pump shaft within the through bore.

12. The suction and irrigation system of claim 11, wherein the second housing portion includes an annular inner wall and the turbine wheel defines a cylindrical bore, the annular inner wall being received within the cylindrical bore to rotatably support the turbine wheel on the second housing portion.

13. The suction and irrigation system of claim 12, wherein the dual impeller pump includes a bushing positioned about the annular inner wall of the second housing portion between the turbine wheel and the annular inner wall.

14. A vacuum assisted suction and irrigation system comprising:
a suction and irrigation wand including a proximal body portion supporting a suction valve and an irrigation valve and a distal body portion defining a fluid channel;
an irrigation fluid supply connected to the irrigation valve of the suction and irrigation wand, the irrigation valve actuable to deliver irrigation fluid to the fluid channel of the suction and irrigation wand;
a vacuum source connected to the suction valve of the suction and irrigation wand, the suction valve actuable to draw a vacuum within the fluid channel; and
a dual impeller pump connected to the irrigation fluid supply and to the vacuum source to deliver pressurized irrigation fluid from the irrigation fluid supply to the suction and irrigation wand, the dual impeller pump including a pump impeller and a turbine wheel, the vacuum source driving the turbine wheel to drive the pump impeller, wherein the irrigation fluid supply is positioned above the first pump chamber such that irrigation fluid from the irrigation fluid supply forces air from the first pump chamber to prime the dual impeller pump.

15. A vacuum assisted suction and irrigation system comprising:
a suction and irrigation wand including a proximal body portion supporting a suction valve and an irrigation valve and a distal body portion defining a fluid channel;
an irrigation fluid supply connected to the irrigation valve of the suction and irrigation wand, the irrigation valve being actuable to deliver irrigation fluid to the fluid channel of the suction and irrigation wand;
a vacuum source connected to the suction valve of the suction and irrigation wand, the suction valve being actuable to draw a vacuum within the fluid channel; and
a dual impeller pump configured to deliver pressurized irrigation fluid from the irrigation fluid supply to the suction and irrigation wand, the dual impeller pump including a housing defining first and second pump chambers, a pump impeller, and a turbine wheel, the pump impeller positioned within the first pump chamber and the turbine wheel positioned within the second pump chamber, the dual impeller pump connected to the vacuum source to drive the pump impeller and the turbine wheel;
wherein the first pump chamber includes a fluid inlet port and a fluid outlet port, and the second pump chamber includes an air inlet port and an air outlet port, the air inlet port defining an inlet channel that communicates with the second pump chamber, the inlet channel having a diameter that converges towards the second pump chamber.
